# EUROPÄISCHE PATENTSCHRIFT

(11) **EP 1 736 121 B1**
(45) Veröffentlichungstag und Bekanntmachung des Hinweises auf die Patenterteilung: **23.07.2008**
(21) Anmeldenummer: 06009499.2
(22) Anmeldetag: 09.05.2006
(51) Int. Cl.: A61F 2/64

(54) **Hydraulische Kniegelenkprothese**
Hydraulic knee joint prosthesis
Prothèse de genou articulée hydraulique

(30) Priorität: 23.06.2005 DE 102005029160
(43) Veröffentlichungstag der Anmeldung: 27.12.2006
(73) Patentinhaber: Dr. Ing. h.c. F. Porsche Aktiengesellschaft, 70435 Stuttgart (DE)
(72) Erfinder: Baumann, Hans-Uwe, 70437 Stuttgart (DE); Kluge, Andreas, 71272 Renningen (DE); Kaschuba, Michael, 71272 Renningen (DE)

(56) Entgegenhaltungen:
- EP-A- 0 654 254
- WO-A-99/00075
- DE-C1- 19 506 426
- US-A1- 2004 186 591

## Beschreibung

Die vorliegende Erfindung betrifft eine Kniegelenkprothese, wie sie in einer Beinprothese zur Adaption an einen Oberschenkelstumpf zum Einsatz kommt und bereits aus der DE 195 06 426 C1 bekannt ist.

Bei dieser bekannten Anordnung wird ein sogenanntes Bremskniegelenk verwendet, welches einem Gelenkoberteil, einem Gelenkunterteil, einer drehfest mit einem Gelenkteil verbundenen Gelenkachse und einem ein Gelenkmittelteil bildenden Schwinghebel, der mit seinem streckseitigen Ende an einer parallel ventral und distal zur Gelenkachse liegenden Schwingachse festgelegt ist und mit seinem beugeseitigen Ende die Gelenkachse umschließt, und wobei mit einer Erfassung der Fußbelastung die Bremseinrichtung gesteuert wird. Hieraus ist es bereits bekannt, die Gelenkachse in einer mit Hydrauliköl befüllten Verdrängerkammer anzuordnen, wobei diese Verdrängerkammer in eine Streckkammer und eine Beugekammer unterteilt sind, die über eine Ölleitung miteinander verbunden sind, welche durch einen Ventilkolben ganz oder teilweise verschließbar ist.

Ausgehend vom bekannten Stand der Technik war es die Aufgabe, die hydraulische Kniegelenkfunktion für eine Beinprothese konstruktiv zu verbessern und in ihrer Ansteuerbarkeit noch näher an den natürlichen Bewegungsablauf beim Gehen, Laufen, Treppen steigen, Radfahren oder sonstige verschiedene Bewegungsabläufe anzupassen.

Mit der erfindungsgemäßen Vorrichtung wurde eine rein hydraulisch arbeitende Kniegelenkprothese für Oberschenkelamputierte geschaffen, die die normalen Lebensbereiche des Trägers bestmöglich abdeckt und beim Tragen im häuslichen Umfeld, im Beruf, in der Freizeit und für sportliche Aktivitäten, wie Wandern, Radfahren, einsetzbar ist. Dabei sind die Funktionen freie Beweglichkeit des unbelasteten Gelenkes, ein Stabilisieren des Bremsen beim Stehen, das Gehen mit variabler Geschwindigkeit, die Unterstützungen der Extensionseinleitungen, also der Schwungaufnahme, ein definierbarer zeitabhängiger Sinkwiderstand beim Treppauf- und Treppabgehen, sowie das Rampenauf- und -abwärtsgehen sowie das Hinsetzen und Aufstehen und Hinknien der Person möglich.

Die hydraulische Kniegelenkprothese ist in der Fig. dargestellt und soll im Folgenden näher erläutert werden.

Die Kniegelenkprothese besteht aus einer Gelenkeinheit G, einer Fußanschlusseinheit F, sowie dem die Komponenten verbindenden Rohr R. Die Gelenkeinheit G beinhaltet eine Drehachse 10, einen die Gelenkeinheit beaufschlagenden Drehdämpfer, sowie dessen aufwendige Hydrauliksteuerung für Aufstandsbremsungen, Regelungen und zeitabhängige Steuerungen, welche im Weiteren noch erläutert werden soll. Die Kniegelenkprothese G umfasst ferner einen Anschlussadapter AS für den Anschluss des Oberschenkelstumpfes und einen Anschlussadapter AF für den Anschluss der Fußprothese.

Die Drehachse 10 der Kniegelenkprothese liegt in der natürlichen Höhe der Kniegelenkachse. Die Kniegelenkachse 10 selbst liegt in einem geschlossenen und mit Hydraulik befüllten Dämpferraum 13. Die Gelenkachse liegt in einem augenförmigen Achslager 11, welches einen Flügel 12 aufweist, der sich mit der Bewegung des Kniegelenkes in der mit Hydrauliköl befüllten Dämpferkammer 13 entsprechend bewegt. Dieses Zusammenspiel sorgt für die Bremswirkung im Kniegelenk. Zur Einstellung verschiedener Bewegungsmodi ist eine hydraulische Steuerung vorgesehen, die die Bewegung der Drehachse 10 in dem augenförmigen Achslager 11 steuert.

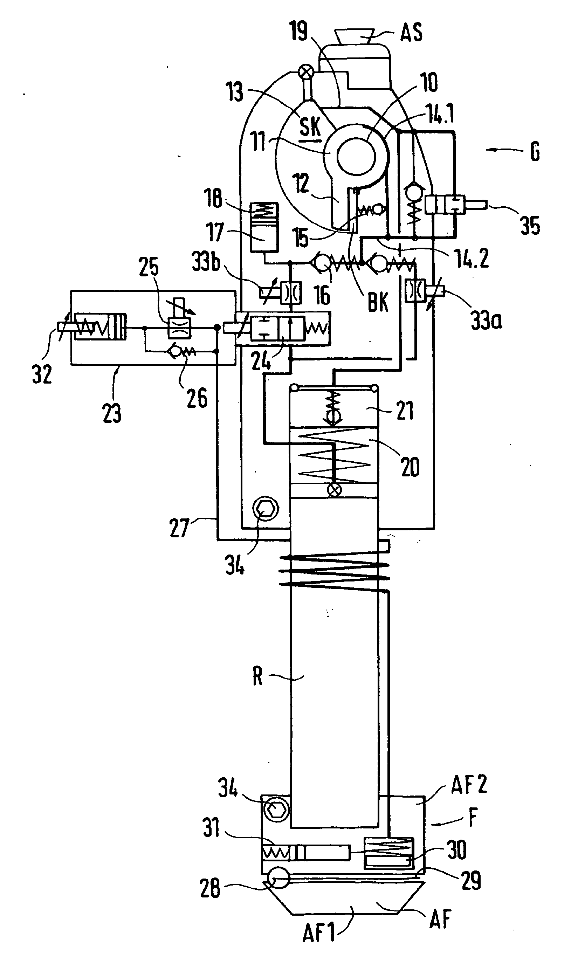

Während der Bewegung des Kniegelenkes verändern sich die einzelnen Abschnitte in der Dämpferkammer 13. Bei gestrecktem Bein, so wie in der Fig. 1 dargestellt, hat die im Folgenden bezeichnete Streckkammer SK ein maximale Größe und der im Folgenden als Beugekammer bezeichnete Abschnitt eine minimale Größe. Bewegt sich nun der Flügel 12 während der Beugung des Kniegelenkes, verändern sich die Größen der Streckkammer SK und der Beugekammer BK derart, dass bei einer maximalen Beugung die Streckkammer SK ihre minimale Größe hat und die Beugekammer BK das maximale Volumen der Dämpferkammer einnimmt.
Während des Beugens des Kniegelenkes aus dem gestreckten Zustand heraus wird Hydraulikflüssigkeit über einen ölführenden Steuerkanal 14, der in der Achslagerung angeordnet ist und den Zu- und Ablauf des Ölflusses in der Dämpferkammer 13 gewährleistet, geführt. Dieser ölführende Steuerkanal 14 kann in einen ersten Abschnitt 14.1 und einem zweiten Abschnitt 14.2 aufgeteilt werden. Die Abstimmung des Ölflusses durch den Steuerkanal 14 erfolgt über dessen Formgebung und Konturen der Achslagerung und den sich dadurch und in Winkelposition ergebenden Öffnungsquerschnitten.
Zwischen dem Abschnitt 14.1 und dem Abschnitt 14.2 des Steuerkanals 14 ist ein Rückschlagventil 15 angeordnet, das zurückführt in die Beugekammer BK der Dämpferkammer 13. Dieses Rückschlagventil 15 realisiert die Umgehung eines verschlossenen hydraulischen Steuerkanals, um ohne Widerstand eine Gegenbewegung des Knies wieder in die Strecklage einleiten zu können. Während der Beugung des Knies und der Bewegung des Dämpferflügels 12 innerhalb der Dämpferkammer 13 wird das hydraulische Öl aus der Streckkammer SK über die Leitungen 14.1 und 14.2 in die Beugekammer BK und parallel dazu über ein Rückschlagventil 16 in eine Ausgleichskammer 17 geleitet. Die Ausgleichskammer 17 weist einen federbeaufschlagten Kolben 18 auf, wodurch Ölschwankungen infolge von Volumenänderungen durch Temperatur ausgeglichen werden können. Die Ausgleichskammer 17 dient ferner dem Ausgleich von Leckölverlusten und sichert zusammen mit dem federbelasteten Kolben 18 den Ölvorrat zum Aufbau einer Überdrucksituation, um ein Eindringen von Luft in das Gesamtsystem zu vermeiden.

Mit der eben beschriebenen Funktion ist das hydraulische Kniegelenk in seiner Funktion beschrieben. Zusammenfassend ist hier nochmals auszuführen, dass das Kniegelenk mit der hydraulischen Bremse im Wesentlichen aus der Drehachse dem augenförmigen Achslager 11 mit dem Dämpferflügel, der Dämpferkammer 13 und dem hydraulischen Steuerkanal 14, welcher im Achslager angeordnet ist, und das Öl aus der Streckkammer SK in die Beugekammer BK bei der Bewegung des Dämpferflügels ableitet, realisiert ist.

Die im Folgenden beschriebenen Ansteuerungen sichern die verschiedenen Funktionsmodi des hydraulischen Kniegelenkes.

Nach der Beugung des Kniegelenkes ist sicherzustellen, dass auch eine Streckung einfach zu realisieren ist. Hierzu ist die Streckkammer SK über eine weitere hydraulisch Leitung 19 mit einem hydraulischen Federspeicher 20 verbunden, der durch das Umlauföl der Dämpferkammer 13 beaufschlagt wird, um die bei der Beugung abgegebene Energie für die Bewegungsumkehr wieder freizusetzen. Der Federspeicher 20 weist einen Kolben auf, welcher beidseitig mit Öl beaufschlagt wird und sich hydraulisch mittig zwischen den Ölräumen vor und hinter dem Dämpferflügel 12 befindet, also zwischen Streckkammer und Beugekammer befindet. Der Kolben 21 weist ferner ein integriertes Rückschlagventil 22 auf, um bei der Streckung auch im Falle von Ölverlusten immer gesichert in die Ausgangsposition zu gelangen.

Ein in die Hydraulikleitung 19 integriertes Umschaltventil ist von außen für den Träger zugänglich und ermöglicht das Umschalten der Funktion von Gehen auf Radfahren.

Der gesamte hydraulische Bereich wird von einer Zentralventileinheit 23 gesteuert. Diese Zentralventileinheit 23 besteht aus einem Zentralventil 24 mit einer einstellbaren Drossel 25, einem parallel zur Drossel 25 angeordneten Rückschlagventil 26 und einer zwischen dem Zentralventil 24 und der Parallelschaltung aus einstellbarer Drossel 25 und Rückschlagventil 26 angreifenden Steuerleitung 27, die direkt mit der Fußadaptereinheit AF verbunden ist und von dieser beaufschlagt wird.

Die Fußanschlusseinheit F mit dem Anschlussadapter für den Fuß AF besteht aus einem zweiteiligen Zusammenbau, wobei die beiden Teile zueinander kinematisch definiert gelagert sind und die Lagerachse mit dem Bezugszeichen 28 bezeichnet ist. Des Weiteren ist zwischen den beiden Teilen des Anschlussadapters Fuß AF eine Elastomereinlage 29 angeordnet. Beim Aufsetzen des Fußes wird je nach Art und Weise, wie der Fuß aufgesetzt wird, ein Auslenkwinkel zwischen den beiden Teilen des Fußadapters bewirkt, welcher eine Versatzbewegung in einem Druckkolben 30 zur Auslösung eines Steuersignals über die Steuerleitung 27 bewirkt. Unterschieden werden hierbei folgende Varianten.

Die Fußanschlusseinheit ist so ausgelegt, dass ein Auftreten mit dem Fuß bodennah als solches erkannt wird und ein hydraulisches Signal über die Steuerleitung 27 zum Sperren der Dämpferkammer 13 ausgelöst wird.

Bei einem Abrollen des Fußes erkennt dies die Steuerhydraulik und öffnet die Dämpferkammer 13.

Die Kinematik, die Lagerung und die Auslegung der Fußanschlusseinheit F, sowie der hydraulischen Übersetzungsverhältnisse des gesamten Steuerkreises sind bei der erfindungsgemäßen Anordnung so ausgelegt, dass die erforderlichen Auslösewege und/oder Winkel an der Fußanschlusseinheit F möglichst gering sind. Die Lagerung und Verbindung der Fußanschlusseinheit F mittels den Elastomereinlagen 29 oder einem aufvulkanisierten Gummi bewirkt, dass die beiden Teile AF1 und AF2 der Fußanschlusseinheit F zueinander positioniert und akustisch getrennt werden. Des Weiteren wird durch die Elastomereinlage 29 ein definierter Auslöseweg und/oder Winkel zueinander ermöglicht und diese durch Elastizitäten unbelastet wieder in die Ausgangsposition zurückpositioniert.
In der Anschlusseinheit des Fußes F ist ferner noch eine Ausgleichskammer mit Kolben vorgesehen zur Aufnahme von Volumenschwankungen des Hydrauliköls infolge von Volumenänderungen durch Temperaturveränderungen. Diese Ausgleichskammer ist in der Figur mit dem Bezugszeichen 31 versehen.
Der gesamte Bewegungsablauf wird durch die Zentralventileinheit 23 gesteuert. Diese Zentralventileinheit 23 ist eine zeitabhängig arbeitende Vorrichtung, wobei nach einem Auslesesignal z.B. durch Fußauftritt nach kurzer Zeit wieder teilweise oder vollständig die Dämpferkreise zu öffnen sind, um so kurzfristig eine hohe und eine sich anschließende niedere Dämpfwirkung zu erzielen. Damit wird das Auftreten kurz maximal gesichert und sofort anschließend eine definierte Teilbeweglichkeit zugelassen, die beispielsweise an einer Treppenstufe erforderlich wird, wenn beim Auftreten auf die Stufe sowie dem anschließenden Drucksinken des Gelenkes ein Erreichen der nächsten Stufe gewährleistet werden soll. Dieses im Anschlussadapter fußerfasste Signal, welches den Druckkolben 30 beaufschlagt, ist über die Steuerleitung 27 an die Zentralventileinheit 23 geführt.

Die Zentralventileinheit 23 enthält des Weiteren einen verstellbaren Wegbegrenzer 32, welcher manuell von der betroffenen Person eingestellt werden kann. Dieser Wegbegrenzer ermöglicht es der Person, den Sinkwiderstand der Kniegelenkprothese genau auf seine Bedürfnisse abgestimmt einzustellen.

Zwei weitere einstellbare Drosseln sind mit den Bezugszeichen 33a und 33b versehen und ermöglichen die getrennte Einstellung der Beugung und Streckung, da sie direkt auf den Öldurchfluss eingreift, der bei der Bewegung aus der Verdrängerkammer 13 abfließt.

Das Rohr R zur Verbindung von Gelenkteil und Fußteil ist jeweils mit einer Klemmung 34 mit der Gelenkeinheit G und der Fußanschlußeinheit AF verbunden. Die Länge des Rohres ermöglicht eine genaue Einstellung der Höhe der Gelenkachse 10 an die Größe des Trägers.

Die einzelnen Bauteile sind oberflächenbehandelt, wobei durch Harteloxieren die allgemeinen Festigkeit verbessert wurde. Die Oberflächenbehandlung aller betroffenen Komponenten wie Hydraulikblock, Dämpferflügel, Ventilkomponenten, Kolben mit eingelagerten Teflon dient zur Optimierung der Gleit- und Verschleißeigenschaften. Des Weiteren sind die Ventilteile zur Gewichtsminimierung aus Leichtmetall gefertigt.

Mit der vorbeschriebenen Kniegelenkprothese und der hydraulischen Ansteuerung der Kniegelenkprothese ist jede für den Nutzer notwendige Bewegungsform optimal abgedeckt und die Prothese somit vielfältig in allen Bereichen des täglichen Lebens nutzbar.

Das Gesamtsystem hat durch seinen Aufbau einen direkt in den Ölkreislauf integrierten Federspeicher, über den die hier eingeleitetet Energie wieder freigesetzt werden kann und so dem Gesamtsystem erhalten bleibt.

## Patentansprüche

1. Hydraulische Kniegelenkprothese für eine Beinprothese mit einer Gelenkeinheit (G), einer Fußanschlusseinheit (F) und einem beide Komponenten verbindenden Rohr (R), wobei die Gelenkeinheit (G) eine Drehachse (10) mit einer Dämpferkammer (13) und einem darin bewegbaren Dämpferflügel (12) beinhaltet, die von einer Hydrauliksteuerung angesteuert ist, **dadurch gekennzeichnet, dass** die Fußanschlusseinheit (F) über eine Steuerleitung (27) mit einer Zentralventileinheit (23) verbunden ist und über Druckpunkte in der Fußanschlusseinheit (F) eine Druckkammer (30) die Steuerleitung (27) zur Ansteuerung der Zentralventileinheit (23) beaufschlagt.

2. Hydraulische Kniegelenkprothese nach Anspruch 1, **dadurch gekennzeichnet, dass** die Zentralventileinheit (23) durch Ansteuerung verschiedener Rückschlagventile die Dämpfer- und Bremswirkung der Dämpferkammer (13) beeinflusst.

3. Hydraulische Kniegelenkprothese nach einem der Ansprüche 1 oder 2, **dadurch gekennzeichnet, dass** einstellbare Drosseln (33) und einstellbare Wegbegrenzer (32) vorgesehen sind, die eine manuelle Beeinflussung der Dämpfereigenschaften der Kniegelenkprothese ermöglichen.

## Claims

1. Hydraulic knee-joint prosthesis for a prosthetic leg, with a joint unit (G), a foot attachment unit (F), and a tube (R) connecting both components, the joint unit (G) having a rotation axle (10) with a damper chamber (13) and a damper wing (12) movable therein, activated by a hydraulic control, **characterized in that** the foot attachment unit (F) is connected to a central valve unit (23) via a control line (27), and, via pressure points in the foot attachment unit (F), a pressure chamber (30) acts on the control line (27) so as to activate the central valve unit (23).

2. Hydraulic knee-joint prosthesis according to Claim 1, **characterized in that** the central valve unit (23) influences the damper action and brake action of the damper chamber (13) by activating various check valves.

3. Hydraulic knee-joint prosthesis according to one of Claims 1 and 2, **characterized in that** adjustable throttles (33) and adjustable path limiters (32) are provided with which the damper properties of the knee-joint prosthesis can be influenced manually.

## Revendications

1. Prothèse de genou articulée hydraulique pour une prothèse de jambe comprenant une unité d'articulation (G), une unité de connexion au pied (F) et un tube (R) reliant les deux composants, l'unité d'articulation (G) présentant un axe de rotation (10) avec une chambre d'amortissement (13) et un battant d'amortissement (12) déplaçable dans celle-ci, qui est commandée par une commande hydraulique, **caractérisée en ce que** l'unité de connexion au pied (F) est connectée par le biais d'une conduite de commande (27) à une unité de soupape centrale (23), et, par le biais de points de pression dans l'unité de connexion au pied (F), une chambre de pression (30) sollicite la conduite de commande (27) pour la commande de l'unité de soupape centrale (23).

2. Prothèse de genou articulée hydraulique selon la revendication 1, **caractérisée en ce que** l'unité de soupape centrale (23) influence, par la commande de différentes soupapes de non retour, l'effet d'amortissement et de freinage de la chambre d'amortissement (13).

3. Prothèse de genou articulée hydraulique selon l'une quelconque des revendications 1 ou 2, **caractérisée en ce que** l'on prévoit des étranglements ajustables (33) et des limitateurs de course ajustables (32) qui permettent d'influencer manuellement les propriétés d'amortissement de la prothèse de genou articulée.
